# EUROPÄISCHE PATENTSCHRIFT

(11) **EP 0 514 328 B1**
(45) Veröffentlichungstag und Bekanntmachung des Hinweises auf die Patenterteilung: **06.12.1995**
(21) Anmeldenummer: 92810330.8
(22) Anmeldetag: 06.05.1992
(51) Int. Cl.: A61K 31/575

(54) **Chenodeoxycholsäure oder Ursodeoxycholsäure zur Behandlung von Erkrankungen der Atmungsorgane**
Chenodeoxycholic acid or ursodeoxycholic acid for the treatment of diseases of the respiratory organs
L'acide chénodeoxycholique ou l'acide ursodéoxycholique pour le traitement des maladies des organes respiratoires

(30) Priorität: 15.05.1991 CH 1452/91
(43) Veröffentlichungstag der Anmeldung: 19.11.1992
(73) Patentinhaber: MEDICHEMIE AG, CH-4107 Ettingen/Basel (CH)
(72) Erfinder: Widauer, Josef Olaf, Dr. med., CH-4123 Allschwil (CH)
(74) Vertreter: Lauer, Joachim, Dr.

(56) Entgegenhaltungen:
- THE MERCK INDEX, 11. Ausgabe, 1989, Merck & Co. Inc., Rahway, NJ (US); S. BUDAVARI et al., Seite 315, Nr. 2044
- THE MERCK INDEX, 11. Ausgabe, 1989, Merck & Co. Inc., Rahway, NY (US); S. BUDAVARI et al., Seite 1556, Nr. 9801
- SCANDINAVIAN JOURNAL OF RHEUMATOLOGY, Band 4, 1975; A. BRUUSGAARD et al., Seiten 169-173

## Beschreibung

### Technisches Gebiet

Die vorliegende Erfindung betrifft die Verwendung der Gallensäuren Chenodeoxycholsäure und Ursodeoxycholsäure oder einer Kombination davon zur Herstellung eines Arzneimittels.

Die chemische Bezeichnung der Chenodeoxycholsäure ist 3α,7α-Dihydroxy-5β-cholan-24-säure. Die Summenformel lautet C24H40O4. Die Strukturformel ist 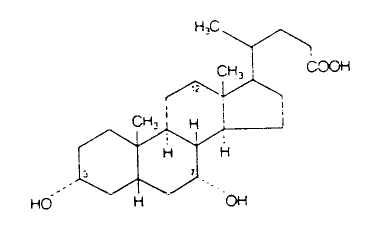
Die chemische Bezeichnung der Ursodeoxycholsäure ist 3α,7α-Dihydroxy-5β-cholan-24-säure. Die Summenformel lautet C24H40O4. Die Strukturformel ist

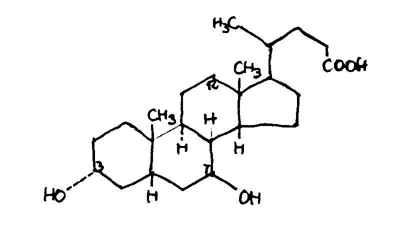

Beide Gallensäuren lassen sich auf halbsynthetischem Wege aus Rindergalle gewinnen.

### Stand der Technik

Es ist bekannt, die vorgenannten Gallensäuren in Tagesdosen zwischen 5 - 20 mg/kg Körpergewicht zur Auflösung von Cholesterin-Gallensteinen sowie bei biliärer Dyspepsie zu verabreichen. Dabei kommen die genannten Gallensäuren entweder einzeln oder in Kombination miteinander zur Anwendung. Aufgrund etwas unterschiedlicher Wirkungsmechanismen entfalten sie im Mischpräparat einen additiven Effekt. Die Ursodeoxycholsäure wird daneben auch zur Behandlung von Refluxgastritis sowie von chronischen Hepatophatien verwendet. Bezüglich dieser bekannten Indikationen kann z.B. verwiesen werden auf Ulrich Leuschner: "Aktuelle Aspekte der Therapie mit Gallensäuren, Deutsches Ärtzeblatt 86, Heft 48, C-2180 - 2186, Nov. 89 oder auf Alan F. Hofmann, " Medical Dissolution of Gallstones by Oral Bile Acid Therapy", The American Journal of Surgery, Volume 158/Number 3, Page 79 - 85, Sept. 89.

### Darstellung der Erfindung

Wie nun überraschenderweise gefunden wurde, sind die genannten Gallensäuren auch bei akuten oder chronischen entzündlichen Erkrankungen der Atmungsorgane, insbesondere der oberen Atemwege, wie z.B. bei chronisch obstruktiver Bronchits, bei chronischer Pharyngits oder auch bei chronischer Tonsillitis therapeutisch gut wirksam.

So wurden in einem klinischen Versuch beispielsweise an drei männliche Patienten im Alter zwischen 53 und 68 Jahren, bei denen die Diagnose einheitlich auf chronische Asthmabronchitis lautete, über drei Wochen täglich drei Kapseln Ursodeoxycholsäure zu je 250 mg verabreicht. Dies entspricht nach dem Körpergewicht der Patienten ca. 10 mg/kg. Die Parameter des Versuchs vor und nach der Behandlung sind aus der nachstehenden Tabelle zu ersehen.

| **subj. und objekt. Prüfungsparameter (n=3)** | **Beurteilung und Gradierung** | |
|---|---|---|
| | **vor Urso-Beh.** | **n. Urso-Beh.** |
| Reizhusten | +++ | + |
| Auswurf | +++ | + |
| Anstrengungsdyspnoe | ++ | + |
| pfeifende Geräusche bei Auskultation | ++ | + |
| path.Spirometrie (vermindertes FEV 1) | ++ | + |
| gestörter pulmonaler Gasaustausch | ++ | + |
| patholog. Sputumbefund | ++ | 0 |
| +++ = stark, ++ = mittel, + = schwach, 0 = fehlend | | |

Danach konnte bei der Kontrolluntersuchung nach drei Wochen Behandlung sowohl subjektiv als auch objektiv eine deutliche Besserung der Atembschwerden festgestellt werden. Die Patienten teilten einstimmig mit, deutlich weniger Reizhusten zu verspüren und vor allem weniger Auswurf zu haben. Auch die Anstrengungsdyspnoe (Atemnot bei Anstrengung) hat sich subjektiv gebessert. Die objekiven Untersuchungsresultate konnten in Übereinstimmung mit den Patientenaussagen die Besserung der Syptome untermauern:
- Bei allen Patienten konnte bei der Spirometrie eine signifikante Besserung des FEV 1= Tiffeneau-Test um mehr als 10% registriert werden (Verbesserung des Atemvolumens).
- Die bronchiale Mischinfektion mit pathogenen Keimen (Haemophilus Ifluenzae, Pneumokokken) in der Voruntersuchung konnte bei der Kontrolluntersuchnung nicht mehr nachgewiesen werden (ohne antibiotische Behandlung). Ebenso verschwunden war die primär deutliche Sputumseosinophilie.
- Schliesslich hat sich auch bei allen Patienten der ärztliche Auskultationsbefund deutlich verbessert.

Gegenstand der vorliegenden Erfindung ist demnach die Verwendung von Chenodeoxycholsäure, von Ursodeoxycholsäure oder einer Kombination von Chenodeoxycholsäure und Ursodeoxycholsäure zur Herstellung eines Arzneimittels zur Behandlung von akuten oder chronischen entzündlichen Erkrankungen der Atmungsorgane.

### Galenische Beispiele

Nachfolgend werden einige galenische Beispiele für die Herstellung von Ursodeoxycholsäure, Chenodeoxycholsäure bzw. eine Kombination aus beiden enthaltenden Kapseln und Filmtabletten angegeben.

### Beispiel 1

### Kapseln

25 Kg Ursodeoxycholsäure werden mit Mais- oder Kartoffelstärke, hochdispersem Siliciumdioxid und Magnesiumstearat gemischt und in 100′000 Hartgelatinekapseln mit einem Gehalt von je 250mg Ursodeoxycholsäure abgefüllt.

### Beispiel 2

### Kapseln

25 Kg Chenodeoxycholsäure werden mit Mais- oder Kartoffelstärke, hochdispersem Siliciumdioxid und Magnesiumstearat gemischt und in 100′000 Hartgelatine Kapseln mit einem Gehalt von je 250mg Ursodeoxycholsäure abgefüllt.

### Beispiel 3

### Kapseln

Je 25 Kg Chenodeoxycholsäure und 25 Kg Ursodeoxycholsäure werden mit Mais- oder Kartoffelstärke, hochdispersem Siliciumdioxid und Magnesiumstearat gemischt und in 100′000 Hartgelatine Kapseln mit einem Gehalt von je 250mg Chenodeoxycholsäure und 250mg Ursodeoxycholsäure abgefüllt.

### Beispiel 4

### Filmtabletten

25 Kg Ursodeoxycholsäure werden mit Mais- oder Kartoffelstärke, hochdispersem Siliciumdioxid, Milchzucker, Zellulosepulver, Magnesiumstearat und Talk zu einem Granulat verarbeitet und zu Tabletten mit einem Gehalt von je 250mg Ursodeoxycholsäure gepresst. Diese Tabletten werden mit einem Film überzogen der aus Polymeren wie Zellulosederivaten, Polymethacrylsäureester, Polyvinylpyrrolidon, Polyethylenglykol besteht.

### Beispiel 5

### Filmtabletten

25 Kg Chenodeoxycholsäure werden mit Mais- oder Kartoffelstärke, hochdispersem Siliciumdioxid, Milchzucker, Zellulosepulver, Magnesiumstearat und Talk zu einem Granulat verarbeitet und zu Tabletten mit einem Gehalt von je 250mg Chenodeoxycholsäure gepresst. Diese Tabletten werden mit einem Film überzogen, der aus Polymeren wie Zellulosederivaten, Polymethacrylsäureester, Poliyvnylpyrrolidon, Polyethylenglykol besteht.

### Beispiel 6

### Filmtabletten

je 25 Kg Chenodeoxycholsäure und 25 Kg Ursodeoxycholsäure werden mit Mais- oder Kartoffelstärke, hochdispersem Siliciumdioxid, Milchzucker, Zellulosepulver, Magnesiumstearat und Talk zu einem Granulat verarbeitet und zu Tabletten mit einem Gehalt von je 250mg Chenodeoxycholsäure und 250mg Ursodeoxycholsäure gepresst.

Diese Tabletten werden mit einem Film überzogen der aus Polymeren wie Zellulosderivaten, Polymethacrylsäureester, Polyvinylpyrrolidon, Polyethylenglykol besteht.

In den vorstehenden Beispielen enthalten die Kapseln und die Filmtabletten mit entweder nur Ursodeoxycholsäure oder nur Chenodeoxycholsäure jeweils 250 mg dieses Wirkstoffes. Bei einer Verabreichung dreimal täglich je einer Tablette bzw. Kapsel entspricht dies bei einer normalgewichtigen Person (75 kg Körpergewicht) etwa der bevorzugten Tagesdosis von 10 mg/kg Körpergewicht. In den Kombinationspräparaten sind 250 mg von beiden Wirkstoffen enthalten. Hierdurch soll auf Grund eines für die erfindungsgemässe Indikation ebenfalls vermuteten, etwas unterschiedlichen Wirkungsmechanismus beider Wirkstoffe ein additiver Effekt erreicht werden.

## Patentansprüche

1. Verwendung von Chenodeoxycholsäure zur Herstellung eines Arzneimittels zur Behandlung von akuten und chronischen entzündlichen Erkrankungen der Atmungsorgane.

2. Verwendung von Ursodeoxycholsäure zur Herstellung eines Arzneimittels zur Behandlung von akuten und chronischen entzündlichen Erkrankungen der Atmungsorgane.

3. Verwendung einer Kombination von Chenodeoxycholsäure und Ursodeoxycholsäure zur Herstellung eines Arzneimittels zur Behandlung von akuten und chronischen entzündlichen Erkrankungen der Atmungsorgane.

## Claims

1. Use of chenodeoxycholic acid for the production of a medicament for the treatment of acute and chronic inflammatory disorders of the respiratory organs.

2. Use of ursodeoxycholic acid for the production of a medicament for the treatment of acute and chronic inflammatory disorders of the respiratory organs.

3. Use of a combination of chenodeoxycholic acid and ursodeoxycholic acid for the production of a medicament for the treatment of acute and chronic inflammatory disorders of the respiratory organs.

## Revendications

1. Utilisation de l'acide chénodéoxycholique pour la préparation d'un médicament pour le traitement des maladies inflammatoires aiguës et chroniques des organes respiratoires.

2. Utilisation de l'acide ursodéoxycholique pour la préparation d'un médicament pour le traitement des maladies inflammatoires aiguës et chroniques des organes respiratoires.

3. Utilisation d'une combinaison de l'acide chénodéoxycholique et de l'acide ursodéoxycholique pour la préparation d'un médicament pour le traitement des maladies inflammatoires aiguës et chroniques des organes respiratoires.
